Europäisches Patentamt

European Patent Office

Office européen des brevets

(19)

(11) Veröffentlichungsnummer: **0 250 000 B1**

(12) # EUROPÄISCHE PATENTSCHRIFT

(45) Veröffentlichungstag der Patentschrift: **11.11.92**

(51) Int. Cl.5: **C12P 21/02**, C12N 15/00, A61K 37/02, C07K 15/00

(21) Anmeldenummer: **87108847.2**

(22) Anmeldetag: **20.06.87**

(54) **Neue Polypeptide, ihre Herstellung und ihre Verwendung.**

(30) Priorität: **20.06.86 DE 3620656**

(43) Veröffentlichungstag der Anmeldung:
**23.12.87 Patentblatt 87/52**

(45) Bekanntmachung des Hinweises auf die
Patenterteilung:
**11.11.92 Patentblatt 92/46**

(84) Benannte Vertragsstaaten:
**AT BE CH DE ES FR GB IT LI NL SE**

(56) Entgegenhaltungen:
**EP-A- 0 164 965**

**THE JOURNAL OF BIOLOGICAL CHEMISTRY,
Band 260, Nr. 4, 25. Februar 1985, Seiten
2334-2344, American Society of Biological
Chemists, Inc., US; B.B. AGGARWAL et al.:
"Primary structure of human lymphotoxin
derived from 1788 lymphoblastoid cell line"**

(73) Patentinhaber: **BASF Aktiengesellschaft
Carl-Bosch-Strasse 38
W-6700 Ludwigshafen(DE)**

(72) Erfinder: **Schollmeier, Klaus, Dr.**

**Landstrasse 80a
W-6944 Hemsbach(DE)**
Erfinder: **Moeller, Achim, Dr.
Wilhelm-Busch-Strasse 51
W-6703 Limburgerhof(DE)**
Erfinder: **Koerwer, Wolfgang, Dr.
Rene-Bohn-Strasse 4
W-6700 Ludwigshafen(DE)**
Erfinder: **Doerper, Thomas, Dr.
Luitpoldstrasse 3
W-6719 Bissersheim(DE)**
Erfinder: **Hillen, Heinz, Dr.
Fasanenstrasse 10
W-6700 Ludwigshafen(DE)**
Erfinder: **Daum, Lothar, Dr.
Reiherstrasse 25
W-6701 Otterstadt(DE)**
Erfinder: **Emling, Franz, Dr.
Valentin-Bauer-Strasse 22
W-6700 Ludwigshafen(DE)**
Erfinder: **Keilhauer, Gerhard, Dr.
Industriestrasse 20
W-6701 Dannstadt-Schauernheim(DE)**

**Beschreibung**

Die Erfindung betrifft neue Polypeptide mit Lymphotoxin-Aktivität oder Lymphotoxin-ähnlicher Aktivität, deren Herstellung und deren Verwendung bei der Bekämpfung von Krankheiten, gegebenenfalls in Kombination mit Lymphokinen.

Lymphotoxin (auch als TNF-beta bezeichnet) wurde 1968 zum ersten Mal beschrieben (Ruddle and Waksman J. exp. Med. 128, 1267-1279 1968; Granger und Kolb J. Immun. 101, 111-120 1968; Rosenau, W. Fed. Proc. 27, 34-38 1968). Lymphotoxin besitzt als biologischer Faktor aus mitogen stimulierten Lymphozyten cytotoxische Aktivität auf neoplastische Zellinien. Es hat ein Spektrum von Aktivitäten, wie Cytostase einiger Tumor-Zellinien und eine deutliche cytolytische Aktivität auf andere transformierte Zellen (Sawada und Osawa Jap. J. exp. Med, 46, 263-267 1976; Granger et al J. Cell. Immun. 38, 388-402 1978; Rundell und Evans Immunopharmacology 3, 9-18 1981; Granger et al J. Lymphokine Res. 1, 45-49 1982; Ruddle et al J. Lymphokine Res. 2, 23-31 1983).

Auf Primärzellkulturen und normale Zellinien zeigt Lymphotoxin weniger oder keine cytotoxische Aktivität. Diese Befunde führten zu in vivo Studien (Khan et al Proc. Soc. exp. Biol. Med. 169, 291-294 1982; Papermaster et al Cancer 45, 1248-1253 1980; Ransom et al J. Natn. Cancer Inst. 69, 741-744 1982), deren Ergebnisse dann zeigten, daß Lymphotoxin ein wirksames Antitumormittel ist.

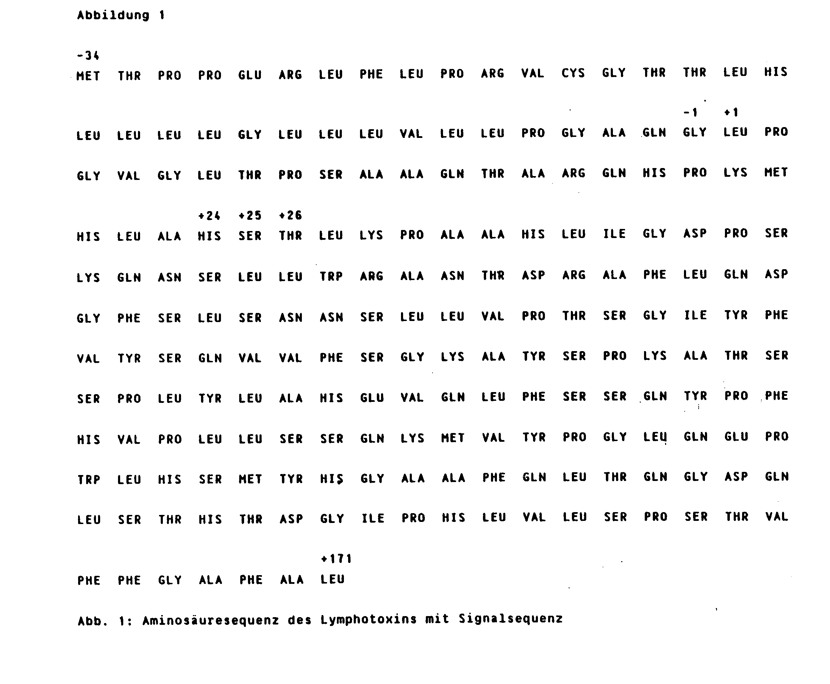

Humanes Lymphotoxin besitzt die in Fig. 1 wiedergegebene Aminosäuresequenz (Gray et al Nature 312, 721-724 1984). Die Signalsequenz des Lymphotoxins ist dabei mit -34 bis -1 gekennzeichnet.

Humanes Lymphotoxin (TNF-beta) gehört zu einer Gruppe von Lymphokinen, zu der auch der Tumor-Nekrose-Faktor (TNF oder TNF-alpha) gehört. Beide Proteine besitzen nicht nur ein ähnliches Wirkungsspektrum in vitro und in vivo sondern wirken auch jeweils synergistisch mit Interferon-gamma (Ruddle et al. Lymphokine Res. 2, 23-31 1983, Granger et al. Lymphokine Res. 1, 45-49 1982; Ruff und Giffort Lymphokines Vol. 2, Pick, E. ed. 235-275 Academic Press New York 1981; Carswell et al. Proc. Natl. Acad. Sci. 72, 3666-3670 1975; Evans Canc. Immunol. Immunother. 12, 181-190 1982; Rundell und Evans Immunopharmacology 3, 9-18 1981; Ruff und Giffort Infect. Immun. 31, 380-385 1981; Williamson et al. Proc. Natl. Acad. Sci. 80, 5397-5401 1983; Williams und Bellanti J. Immunol. 130, 518-520 1983; Stone-Wolff et al. J. Exp. Med. 159, 828-843 1984; Lee et al. J. Immunol. 133, 1083-1086 1984; Powell et al. Lymphokine Res. 4, 13-26 1985).

Die Gene für beide Proteine sind auf dem Chromosom 6 benachbart lokalisiert (Nedwin et al. Nucl. Acid Res. 13, 17; 6361-6373 1985).

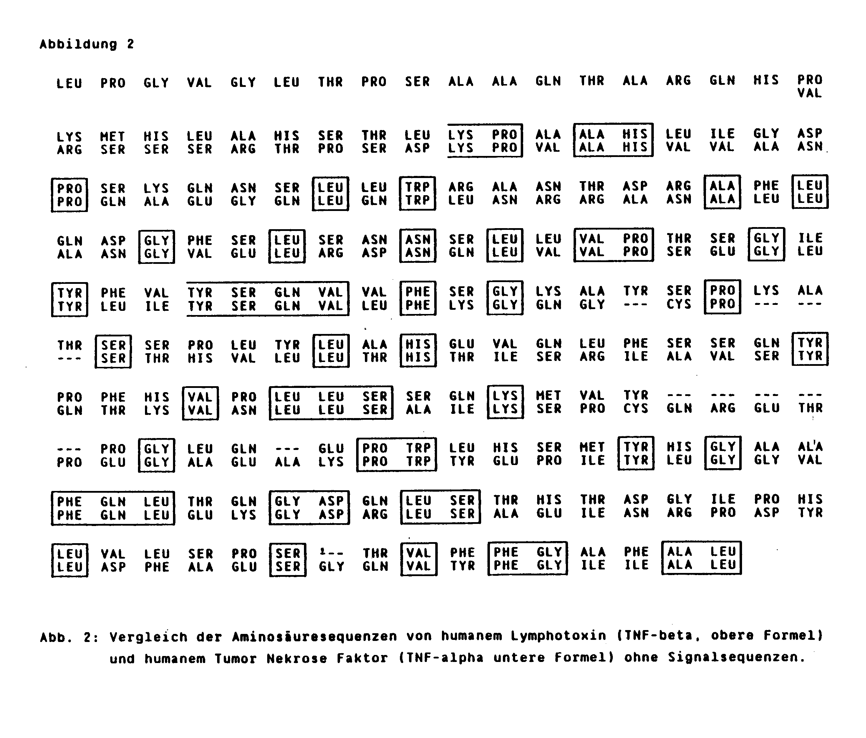

Ein Aminosäurevergleich beider Proteine zeigt, daß sie auf Aminosäureebene eine Homologie von 30 % besitzen (Fig. 2). Die Homologie konzentriert sich dabei auf den mittleren und C-terminalen Teil der beiden Proteine, während der N-Terminus heterolog und unterschiedlich lang ist (Fig. 2).

Weiterhin ist eine Lymphotoxinmutante bekannt, die sich an ihrem N-Terminus von dem natürlichen Lymphotoxin durch das Fehlen der ersten 23 Aminosäuren auszeichnet.

Es wurde nun gefunden, daß Polypeptide, die aus den Aminosäuresequenzen 25-171 und 26-171 des Lymphotoxins bestehen, vorteilhaftere Eigenschaften besitzen.

Die neuen Peptide lassen sich herstellen, indem man

a) aus einer Lymphotoxin produzierenden Zellinie die mRNA isoliert,

b) diese mRNA in die entsprechende doppelsträngige cDNA kopiert,

c) diese cDNA in Vektoren von E.coli insertiert,

d) E.coli mit den so erhaltenen neuen Vektoren transformiert,

e) die Lymphotoxin cDNA Klone mit Hilfe von Gensonden und Hybridisierung selektioniert und charakterisiert,

f) die das Lymphotoxin-Gen enthaltenden Vektoren vermehrt und isoliert,

g) mit Hilfe von Restriktionsendonukleasen das Gen oder die Genfragmente isoliert,

h) das Gen oder die Genfragmente mit geeigneten Oligonukleotiden in Expressionsvektoren insertiert,

i) gegebenenfalls zusätzliche DNA-Sequenzen am 5′-Ende der Gene insertiert,

j) E.coli mit diesen Expressionsvektoren transformiert und

k) das gewünschte Genprodukt exprimiert, isoliert und reinigt.

Für die Isolierung der entsprechenden cDNA wurde die Lymphoblastoid-Zellinie RPM I 1788 (ATCC Nr. CCL 156) wie beschrieben kultiviert (Aggarwal et al. J. Biol. Chem. 259, 686-691 1984), die mRNA nach Stimulierung isoliert und nach bekanntem Verfahren in cDNA übersetzt.

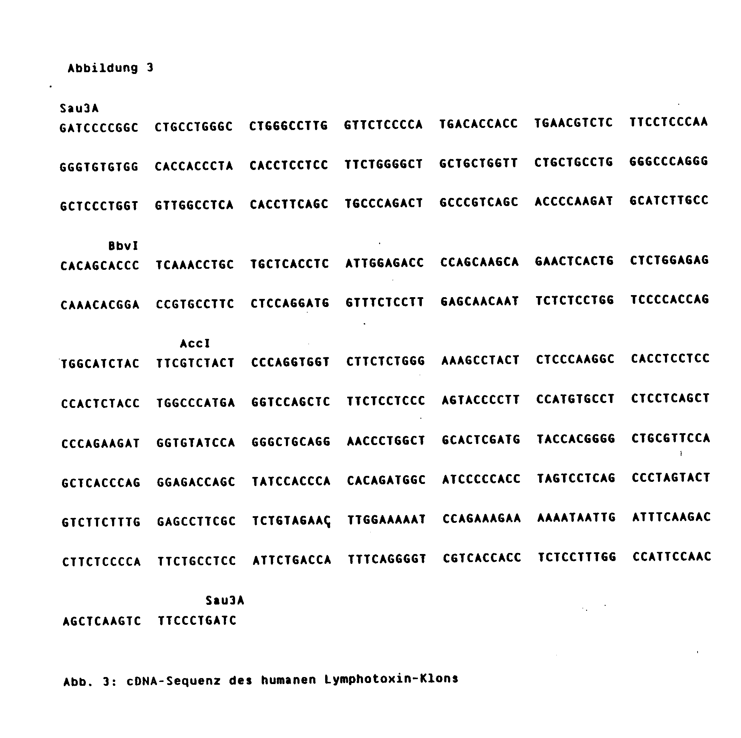

Der cDNA-Klon besitzt dabei die in Fig. 3 wiedergegebene Sequenz. Teile dieser Sequenz, die durch enzymatische Spaltung an Restriktionserkennungsstellen leicht zugänglich sind, werden benutzt, um die in den Beispielen näher beschriebenen neuen lymphotoxinähnlichen Polypeptide zu klonieren. Der Einbau der Genfragmente in Klonierungsvektoren, beispielsweise in die handelsüblichen Plasmide pUC18 und pUC19, erfolgte nach publizierten Methoden (Maniatis et al, Molecular Cloning, Cold Spring Harbor Laboratory 1982). Auch können die Gene oder Genfragmente mit geeigneten chemisch synthetisierten Kontrollregionen versehen werden, die eine Expression der Proteine ermöglichen. Die Transformation der so erhaltenen Hybridplasmide in geeignete Wirtsorganismen, vorzugsweise E. coli, ist ebenfalls bekannt und eingehend beschrieben. Auch können die Hybridplasmide mit entsprechenden Signalsequenzen versehen werden, die die Sekretion der Polypeptide in das Periplasma von E. coli erlauben. Diese so gewonnenen Proteine sind dann an ihrem N-terminalen Ende nach der Sekretion Methionin-frei, besitzen aber meist noch eine für die Spaltstelle wichtige Aminosäure, wie z. B. Alanin (Nucl. Acid. Res. 8, 3011-3027 1980).

Aufgrund der Degeneration des genetischen Codes ist es aber auch möglich, andere DNA-Sequenzen, z. B. chemisch synthetisierte Gene mit unterschiedlicher DNA-Sequenz, für die Expression der neuen Polypeptide zu benutzen.

Die neuen Peptide können bei Tumorerkrankungen, bei Immunkrankheiten oder bei Entzündungskrankheiten wie Rheuma oder Polyarthritis eingesetzt werden.

Die Wirkung der neuen Peptide kann durch Zusatz von Lymphokinen, wie beispielsweise TNF-$\alpha$ und insbesondere Interferon-$\alpha$, Interferon-$\beta$ und Interferon-gamma, sowie von Zugabe von Cancerostatica in überadditiver Weise gesteigert werden. Als Cancerostatica kommen insbesondere in Betracht:

a) Antibiotica wie Actinomycin D, Doxorubicin (Adriamycin), Daunorubicin, Mithramycin und Bleomycin sowie andere interchalatorisch wirkende Substanzen, wie Amonafide und Mitonafide,

b) Alkaloide wie Vincristin, Vinblastin, Vindesin, Etoposid und Teniposid,

c) alkylierend wirkende Substanzen, wie Cyclophosphamid, Nitrosoharnstoffe und Cisplatin und

d) Antimetabolite wie Methotrexat, 5-Fluoruracil und dessen Analoge, 6-Mercaptopurin, 6-Thioguanin und Cytarabin.

Die Erfindung betrifft daher auch Kombinationen der neuen Peptide mit Lymphokinen, wie TNF-$\alpha$ und insbesondere Interferon-$\alpha$, Interferon-$\beta$ und Interferon-gamma, sowie mit Cancerostatica.

Gegenstand der Erfindung sind daher auch Arzneimittel die mindestens eines der neuen Polypeptide enthalten, gegebenenfalls in einem pharmazeutisch verträglichen Träger oder Bindemittel. Weiterhin sind Gegenstand der Erfindung Arzneimittel mit Kombinationen von den neuen Proteinen mit schon bekann ten Lymphokinen oder Lymphokinmutanten (z. B. Interferon-gamma; TNF).

Weitere Ausgestaltungen der Erfindung sind in den folgenden Beispielen näher beschrieben. Das als Vergleichssubstanz dienende Lymphotoxin wurde in analoger Weise zu den Lymphotoxinmutanten in E.coli kloniert, exprimiert und wie beschrieben aufgereinigt.

1. Herstellung der cDNA

Züchtung der Lymphotoxin (TNF-$\beta$) produzierenden Zellinie RPMI-1788

Die haematopoetische Zellinie RPMI-1788 erhalten von ATCC (No. CCL 156) wurde in Spinnerflaschen in RPMI 1640 Medium mit 20 % foetalem Kälberserum bei 37° C und 5 % $CO_2$ kultiviert. Nach Erreichen einer Zelldichte von 7-8 x $10^5$ Zellen/ml wurde das Medium gegen RPMI 1640 Medium mit 5 % foetalem Kälberserum ausgetauscht und der Tumorpromotor PMA (4$\beta$-Phorbol-12$\beta$-myristat-13$\alpha$-acetat) in der zuvor bestimmten optimalen Konzentration von 150 nM hinzugeben. Nach 70 h wurden maximale Mengen an Lymphotoxin (700 U/ml) produziert. Die biologische Aktivität wurde, wie beschrieben, (Aggarwal, B.B.; Moffat, B. und Harkins, R.N. (1984) J. Biol. Chem. Vol 259, 686-691) bestimmt. Nach dieser Zeit wurden die Zellen gesammelt, mit 2 ml PBS gewaschen und in einem Lysepuffer (6 M Guanidiniumthiocyanat, 5mM Natriumcitrat pH 7,0, 0,1 M 2-Mercaptoethanol, 0,5 % Sarkosyl) mit Hilfe eines Homogenisators aufgeschlossen. Die RNA wurde durch ein 5,7 M CsCl-Kissen bei 35000 rpm über Nacht sedimentiert. Die poly $A^+$ enthaltende Fraktion der mRNA wurde durch zweimalige Affinitätschromatographie an oligo (dT) Cellulose isoliert und gereinigt.

Mit Hilfe des Enzyms AMV-Reverse Transkriptase wurde die poly $A^+$ RNA in einsträngige cDNA umgeschrieben. Die Synthese des zweiten Strangs erfolgte ebenfalls mit Reverser Transkriptase. Die doppelsträngige cDNA wurde mit der Restriktionsendonuklease Sau 3A nach Angaben des Herstellers geschnitten und auf einem 1 %igen Agarosegel fraktioniert. Die DNA-Fragmente der Größe 1 kbp $^1$0,5 kbp wurden aus dem Gel eluiert und mit dephosphoriliertem, BamHI-liniearisierten E.coli Vektor pUC 18 ligiert. Die DNA wurde in kompetente E.coli Zellen des Stammes HB 101 transformiert und auf LB-Platten mit 100 $^5$g/ml Ampicillin ausplattiert. Die Bakterien wurden auf Nitrocellulosefilter übertragen, repliziert, lysiert, die DNA, denaturiert und fest an das Filter gebunden.

Mit Hilfe eines DNA-Synthesizers (Applied Biosystems, Typ 380 A) wurden drei 17er Oligonukleotidsonden mit Homologie zur publizierten DNA-Sequenz des humanen-Lymphotoxins hergestellt. Diese Sonden

hatten folgende Sequenzen:

$$5'CCTCCTGCACCTGCTGC\ 3'$$
$$5'TTGCTGGGGTCTCCAAT\ 3'$$
$$5'GAGTGCAGCCAGGGTTC\ 3'$$

Die Oligonukleotidsonden wurden am 5'-Ende mit $\gamma$-$^{32}$P-ATP markiert und die Nitrocellulosefilter in 1 M NaCl, 1 mM EDTA, 1 % SDS, 10 % Dextransulfat über Nacht bei 42°C unter Schütteln mit den Sonden inkubiert. Nach intensivem Waschen der Filter in 1 M NaCl, 1 mM EDTA, 1 % SDS wurden die Filter mit einem Film exponiert und Klone mit Sequenzhomologie ermittelt. Die homologen Klone wurden isoliert und vereinzelt. Die Plasmid DNA wurde isoliert und sequenziert. Einer der so isolierten Klone war pLyt 15, dessen Sequenz in Abb.3, dargestellt ist.

2. Herstellung eines Hybridplasmids, das das Genfragment für die vergleichssubstanz mit der Aminosäuresequenz 24-171 enthält (delta 23 Lymphotoxin).

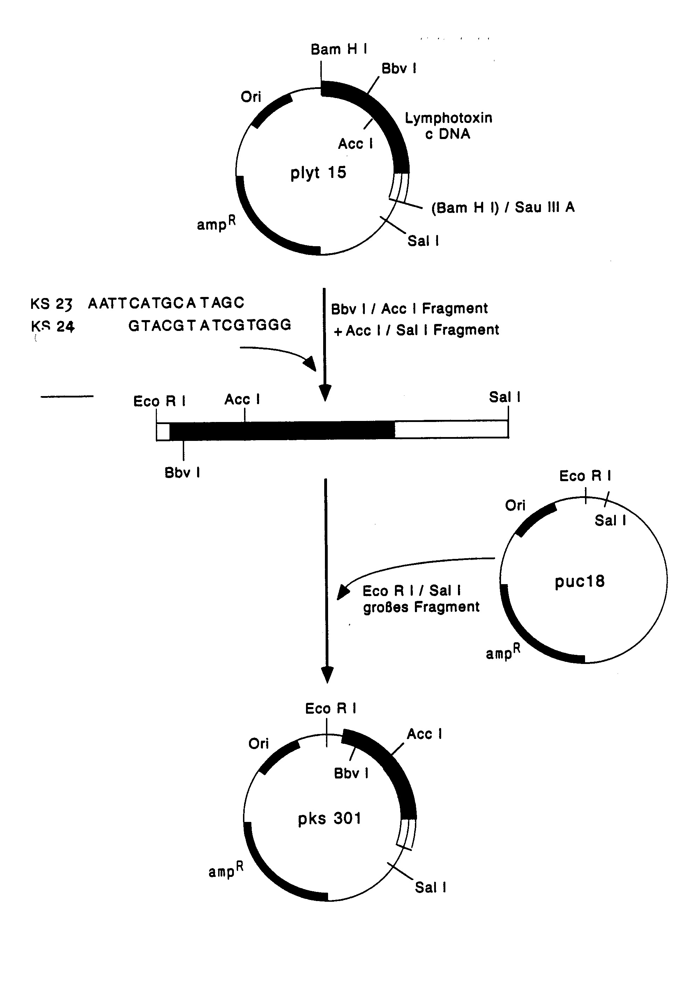

Ausgangspunkt ist das Plasmid pLyt 15. Es entsteht durch die Insertion der mit Sau3A geschnittenen Lymphotoxin cDNA (Fig. 3) in die BamH1 Erkennungsstelle von pUC18. Das Plasmid pLyt 15 wurde in bekannter Weise mit den Restrictionsendonucleasen Bbv1/Acc1 sowie Acc1/Sal1 nach den Angaben des Herstellers geöffnet (Fig. 3 und 4). Der Verdauungsansatz wurde auf einem 5 %igen Polyacrylamidgel durch Elektrophorese in bekannter Weise aufgetrennt und die Bruchstücke durch Anfärben mit Ethidiumbromid sichtbar gemacht. Die beiden benötigten Lymphotoxin-Genfragmente Bbv1/Acc1 und Acc1/Sal1 wurden anschließend aus dem Acrylamidgel ausgeschnitt en und elektrophoretisch von der Matrix abgetrennt. 0,1 pMol dieser beiden Fragmente wurden dann mit 0,1 pMol Vektor (pUC18 EcoR1/Sal1) und dem synthetischen Oligonukleotid KS 23/24 über Nacht bei 15° C ligiert.

$$KS23:\ 5'AATTCATGCATAGC\ 3'$$
$$KS24:\ 3'\ GTACGTATCGTGGG\ 3'$$

Man erhielt das Hybridplasmid pKS301 gemäß Fig. 4.

3. Herstellung eines Hybridplasmids, das das Genfragment für das Polypeptid mit der Aminosäuresequenz 25-171 enthält (delta 24 Lymphotoxin).

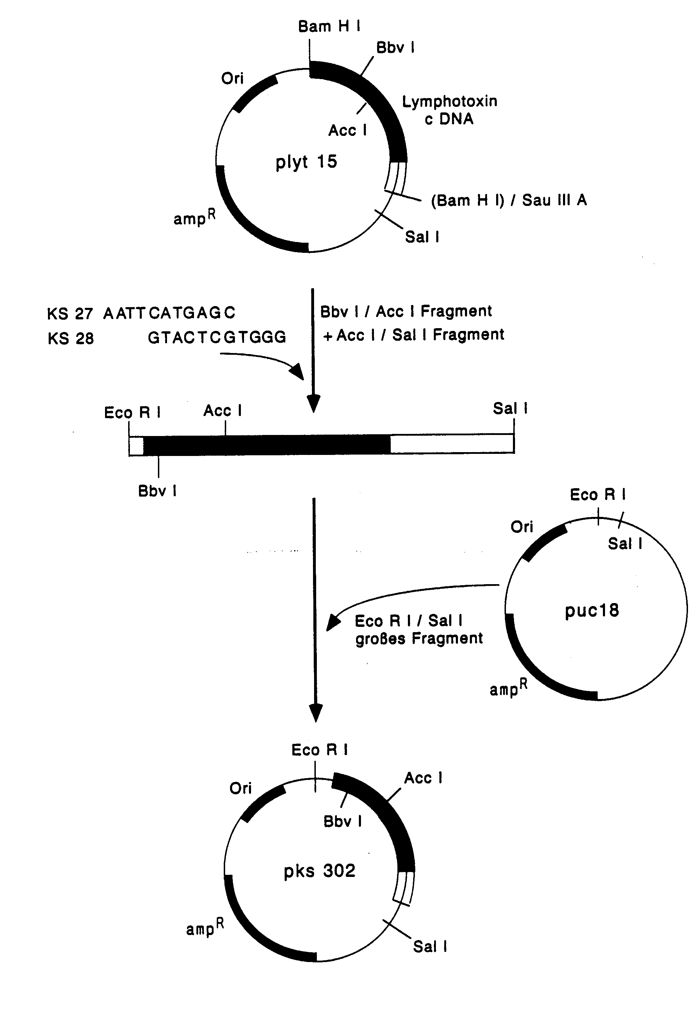

Ausgangspunkt ist das Plasmid pLyt 15. Es entsteht durch die Insertion der mit Sau3A geschnittenen Lymphotoxin cDNA (Fig. 3) in die BamH1 Erkennungsstelle von pUC18. Das Plasmid pLyt 15 wurde in bekannter Weise mit den Restrictionsendonucleasen Bbv1/Acc1 sowie Acc1/Sal1 nach den Angaben des Herstellers geöffnet (Fig. 3 und 5). Der Verdauungsansatz wurde auf einem 5 %igen Polyacrylamidgel durch Elektrophorese in bekannter Weise aufgetrennt. Die Bruchstücke wurden durch Anfärben mit Ethidiumbromid sichtbar gemacht. Die beiden benötigten Lymphotoxin-Genfragmente Bbv1/Acc1 und Acc1/Sal1 wurden anschließend aus dem Acrylamidgel ausgeschnitten und elektrophoretisch von der Matrix abgetrennt. 0,1 pMol dieser beiden Fragmente wurden dann mit 0,1 pMol Vektor (pUC18 EcoR1/Sal1) und dem synthetischen Oligonukleotid KS 27/28 über Nacht bei 15°C ligiert.

$$KS27:\ 5'\ AATTCATGAGC\ 3'$$
$$KS28:\ 3'\ GTACTCGTGGG\ 5'$$

Man erhielt das Hybridplasmid pKS302 gemäß Fig. 5.

4. Herstellung von Hybridplasmiden, die die Genfragmente für das Polypeptid mit der Aminosäuresequenz

4

26-171 enthalten (delta 25 Lymphotoxin).

Die Konstruktion des Hybridplasmids erfolgte wie in Beispiel 2 und 3 beschrieben mit den neuen Oligonukleotiden KS29/30.

$$KS29: \; 5' \; AATTCATG3'$$
$$KS30: \; 3' \; GTACTGGG_5'$$

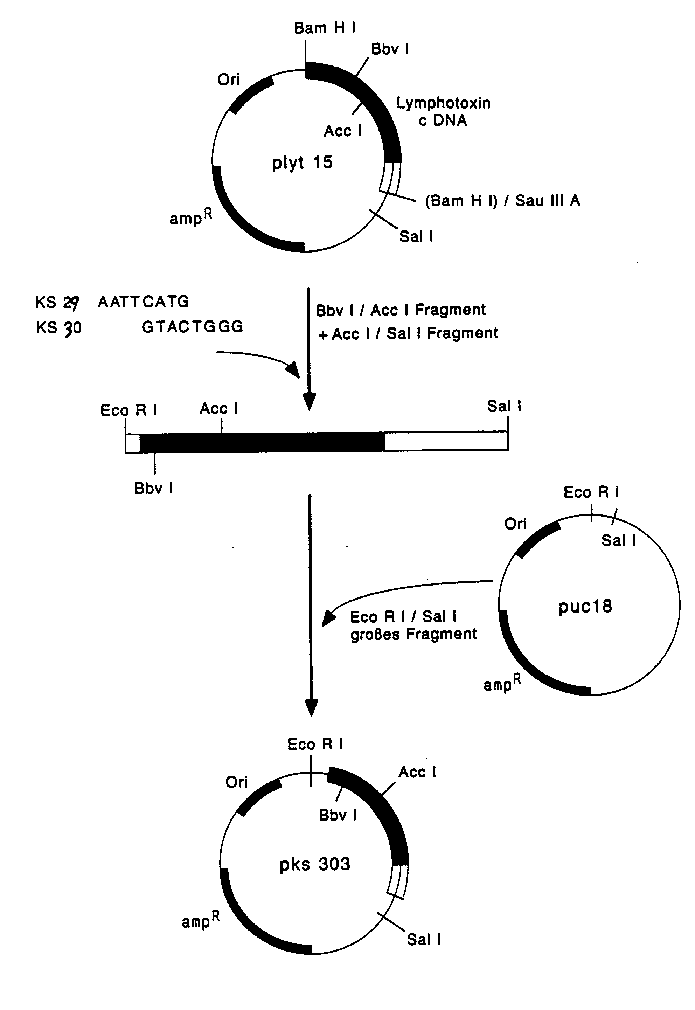

Man erhielt analog Beispiel 2 und 3 das neue Hybridplasmid KS303 (siehe Fig. 6).

5. Transformation der Hybridplasmide

Kompetente E.coli Zellen z.B. W3110 (ATCC 27325) wurden mit 0,1-1 $\mu$g der Hybridplasmide pKS301, pKS302 und pKS303 transformiert und auf Ampicillin enthaltenden Agarplatten ausplattiert. Anschließend ließen sich Klone, die korrekt integrierte Lymphotoxinteilsequenzen enthalten, durch Plasmidschnellanalysen identifizieren.

6. Expression der Lymphotoxinaktivität aufweisenden Polypeptide

Die in Beispiel 2, 3 und 4 erhaltenen Hybridplasmide wurden in an sich bekannter Weise in ihrer einzigen EcoR1 Stelle mit synthetisch hergestellten Signalsequenzen für die Expression versehen. Diese neuen Expressionsplasmide wurden in E.coli transformiert. Die Kultivierung der Produktionszellen erfolgte in normalem Vollmedium (10 g/l Pepton; 5 g/l Hefeextrakt, 10 g/l NaCl und 100 mg/l Ampicillin). Für die Produktion des Lymphotoxins und der Lymphotoxinmutanten wurde folgendes Medium verwendet:

| | | |
|---|---|---|
| Hefeextrakt, Difco | 20 | g/l |
| NZ Amine A, Sheffield | 20 | g/l |
| $K_2HPO_4$ | 4 | g/l |
| $KH_2PO_4$ | 1 | g/l |
| $NH_4Cl$ | 1 | g/l |
| $CaCl_2 \cdot 2\,H_2O$ | 0,01 | g/l |
| $MnCl_2 \cdot 4\,H_2O$ | 0,2 | g/l |
| $K_2SO_4$ | 2,6 | g/l |
| $MgSO_4 \cdot 7\,H_2O$ | 0,5 | g/l |
| EDTA | 0,05 | g/l |
| $FeCl_3 \cdot 6\,H_2O$ | 0,005 | g/l |
| ZnO | 0,0005 | g/l |
| $CuCl_2 \cdot 2\,H_2O$ | 0,0001 | g/l |
| $CO(NO_3)_2 \cdot 6\,H_2O$ | 0,0001 | g/l |
| $NH_4$-Molybdat $\cdot 4\,H_2O$ | 0,0001 | g/l |
| + 100 mg/l Ampicillin | | |
| pH Begrenzung auf 7,0 | | |

7. Reinigung und Charakterisierung der Vergleichssubstanz Lymphotoxin

136 g E.coli-Feuchtmasse eines Lymphotoxin produzierenden Stammes (vgl. EP 164 965), die durch Zentrifugation entsprechender Lymphotoxin exprimierender Schüttelkulturen gewonnen wurde, wurde mit 800 ml 20 mM Natriumphosphatpuffer, 400 mM Arginin•HCl, pH 8,5, versetzt. Die Zellsuspension wurde durch Ultraschallbehandlung bei 4°C aufgeschlossen. Die Fällung der Nukleinsäuren erfolgte mit 20 ml 2 M

MnCl$_2$-Lösung unter Justierung des pH-Wertes auf pH 7,2 mit 12,5 %iger Ammoniaklösung. Nach Zentrifugation und Abtrennen des Niederschlags wurde der Überstand mit 390 g Ammoniumsulfat bei 4°C unter Rühren versetzt. Das Proteinpräzipitat wurde nach Zentrifugation und Verwerfen des Überstandes in 200 ml 20 mM Natriumphosphatpuffer 0,1 mM Arginin •HCl, pH 8,5, gelöst. N ach der Dialyse genen diesen Puffer wurden nacheinander Ionenaustauscherchromatographien an Q-Sepharose, S-Sepharose und Mono Q (Fa. Pharmacia) durchgeführt. Die so erhaltene Lymphotoxinlösung enthielt nach ELISA-Bestimmung zwischen 0,01-0,05 % restliche E-coli-Proteine. Die N-terminale Sequenzierung zeigte, daß es sich um ein Gemisch folgender Lymphotoxinderivate handelt:

1) Met-Lymphotoxin Met-Leu-Pro-Gly-Val-Gly-Leu-Thr-Pro-Ser

2) delta 3-Lymphotoxin Val-Gly-Leu-Thr-Pro-Ser-Ala-Ala-Gln-Thr

3) delta-23-Lymphotoxin His-Ser-Thr-Leu-Lys-Pro-Ala-Ala-His-Leu

8. Reinigung und Charakterisierung der Vergleichssubstanz delta 23-Lymphotoxin

20 g abzentrifugierte E.coli-Zellen aus entsprechenden Schüttelkulturen (siehe Beispiel 2 + 6) wurden in 200 ml 20 mM Natriumphosphatpuffer, pH 8,5 aufgenommen und nach 15-minütiger Ultraschallbehandlung mit 4 ml 2 M MnCl$_2$-Lösung zur Fällung der Nukleinsäuren versetzt. Nach Zentrifugation wurde der Überstand mit verdünnter Ammoniaklösung auf pH 8,9 eingestellt und mit 78 g Ammoniumsulfat das Rohprotein gefällt. Der Niederschlag wurde nach Zentrifugation isoliert, in 100 ml 10 mM Natriumphosphatpuffer, pH 9,0, gelöst und gegen diesen Puffer dialysiert. Durch Chromatographie an Q-Sepharose und S-Sepharose (Fa. Pharmacia) wurde eine homogene Proteinlösung erhalten, die N-terminal methioniertes delta 23-Lymphotoxin in einer Reinheit von über 99 % (nach Analyse durch SDS-Gelelektrophorese) enthielt. Das Protein hatte die N-terminale Sequenz Met-His-Ser-Thr-Leu-Lys-Pro-Ala-Ala-His-Leu-Ile.

9. Aufreinigung und Charakterisierung von delta 4-Lymphotoxin

110 g Biofeuchtmasse aus entsprechenden delta 24-Lymphotoxin produzierenden E.coli-Schüttelkulturen (siehe Beispiel 3 + 6) wurden nach Zentri fugation in 1 l Puffer (20 mM Natriumphosphat, 400 mM Arginin-HCl, pH 8,5) suspendiert und durch Ultraschallbehandlung bei 4°C aufgeschlossen. Zur Fällung der Nukleinsäuren wurde die erhaltene Suspension mit 20 ml 2 M MnCl$_2$-Lösung versetzt. Nach 1,5 h wurde die lösliche Proteinfraktion durch Zentrifugation gewonnen. Der pH-Wert dieser Lösung wurde durch verdünnte Ammoniak-Lösung auf pH 8,9 eingestellt. Durch Zugabe von 390 g Ammoniumsulfat (60 % Sättigung) bei 4°C wurde das delta 24-Lymphotoxin unter Rühren gefällt. Der Ammoniumsulfatniederschlag wurde in 300 ml Puffer (20 mM Natriumphosphat, 0,1 mM Arginin-HCl, pH 10,5) gelöst und über Nacht gegen diesen Puffer (20 l) dialysiert. Die dialysierte Proteinlösung wurde über eine Anionenaustauchersäule (Q-Sepharose-ff, Fa. Pharmacia), chromatographiert. Zur Feinreinigung wurde das Protein nacheinander einer Chromatographie an CM-Sepharose (Fa. Pharmacia) und S-Sepharose unterzogen Die Reinheit des Proteins ist nach SDS-Polyacrylamidgelelektrophorese größer als 99 %. Das Protein ist frei von N-terminalem Methionin. Die N-terminale Sequenz lautet Ser-Thr-Leu-Lys-Pro-Ala-Ala-His-Leu-Ile.

10. Reinigung und Charakterisierung von delta 25-Lymphotoxin

176 g Biofeuchtmasse aus delta 25-Lymphotoxin produzierenden E.coli-Schüttelkulturen (siehe Beispiel 4 + 6) wurden nach Zentrifugation mit 600 ml Puffer (20 mM Natriumphosphat, 400 mM Arginin, pH 8,5) suspendiert. Der Zellaufschluß erfolgte durch Ultraschallbehandlung bei 4°C. Zur Fällung der Nukleinsäuren wurde die Suspension mit 2 M MnCl$_2$-Lösung auf einen Endgehalt von 40 mM eingestellt. Gleichzeitig wurde der pH-Wert mit 12,5 %iger Ammoniaklösung auf pH 7,5 eingestellt. Nach Zentrifugation wurde durch Zugabe von 390 g Ammoniumsulfat pro Liter Überstand das delta 25-Lymphotoxin bei 4°C gefällt. Der Niederschlag wurde in 300 ml 20 mM Natriumphosphatpuffer, pH 10,5, gelöst. Zur Entfernung unlöslicher Proteinaggregate wurde die trübe Lösung zentrifugiert. Der Überstand wurde gegen 5 mM Natriumphosphatpuffer, pH 8,5, dialysiert und anschließend über eine Q-Sepharose-Säule (Fa. Pharmacia) chromatographiert. Zur Feinreinigung wurde die delta 25-Lymphotoxin-haltige Fra ktion nacheinander einer Chromatographie an CM-Sepharose (Fa. Pharmacia) und Q-Sepharose (Fa. Pharmacia) unterzogen. Die Reinheit des so erhaltenen Proteins ist nach SDS-Polyacrylamidgelelektrosepharose größer als 99 %. Das Protein enthielt je nach Fermentationsbedingungen nur wenig oder kein N-terminales Methionin, die N-terminale Sequenz wurde zu
Thr-Leu-Lys-Pro-Ala-Ala-His-Leu-Ile-Gly-.... bestimmt.

11. In vitro zytotoxische Aktivität von humanem recombinantem Lymphotoxin (LT) und (LT)-Mutanten delta 23, delta 24, delta 25 gegenüber den TNF-und Lymphotoxin-sensitiven Zellinien L929 und WEHI-164

$5x10^3$ frisch trypsinisierte, im exponentiellen Wachstum sich befindliche Zellen wurden in 96-Loch-Platten in 125 $^5$l komplettem Wachstumsmedium (MEM mit Earle's Salzen + 10 % FCS, Flow Laboratories, Meckenheim, FRC) ausplattiert und über Nacht bei 37° C 5 % $CO_2$ in einer Wasserdampf-gesättigten Atmosphäre inkubiert. Die Substanzzugabe erfolgte am nächsten Tag in 25 $^5$l komplettem Kulturmedium pro Kulturloch. Die Startkonzentration betrug jeweils 10 ng Protein pro ml; titriert wurde seriell 2-fach mit einer Doppelbestimmung. Folgende Kontrollen wurden auf jeder Kulturplatte mitangelegt: a) nur Kulturmedium; b) Zellen mit Kulturmedium, aber ohne Lymphotoxin; c) ein titrierter TNF-Standard mit bekannter biologischer Aktivität. Nach einer weiteren Inkubation von 48 h bei den oben angegebenen Bedingungen wurden die überlebenden Zellen mit einer Kristallviolettlösung (15 g Kristallviolett, 7 g NaCl, 646 ml Ethanol, 172,8 ml 37 %iges Formaldehyd auf 2 l mit $H_2O$ aufgefüllt) angefärbt. Dazu wurden nach dem Abschlagen des Kulturmediums die Zellen für 20 min mit 50 $\mu$l der Färbelösung bei Raumtemperatur gefärbt. Die Kulturplatten wurden danach so lange mit Wasser gewaschen, bis alle nicht zellgebundenen Farbstoffanteile entfernt waren. Der zellgebundene Farbstoff wurde nach Zugabe von 100 $\mu$l Meßlösung (50 % Ethanol, 0,1 % Essigsäure) bei 540 nm mit Hilfe eines Titertek Multiscan MCC/340 (Flow Laboratories, Meckenheim) photometrisch bestimmt. Angegeben wurden die Konzentrationen an Lymphotoxin bzw. LT-Mutanten, die eine 50 %ige Lyse der Zellen im Vergleich zu der unbehandelten Zellkontrolle ergeben (gemessen über die Reduktion der Absorption).

Eine Einheit (U) ist als diejenige Menge an Lymphotoxin oder LT-Mutanten, definiert, die eine 50 %ige Lyse der gegebenen Zellzahl induziert.

Daraus ergeben sich für die Indikatorzellinie L929 folgende biologische Aktivitäten (korrigiert gegen den mitgeführten TNF-Standard mit einer Aktivität von $8,2x10^6$ U/mg Protein):

| Substanz | Aktivität (U/mg Protein) |
|---|---|
| Lymphotoxin (LT) | $6,1x10^7$ |
| delta 23LT | $1,2x10^8$ |
| delta 24LT | $2,0x10^8$ |
| delta 25LT | $8,5x10^7$ |

Gegenüber der Tumorzellinie WEHI-164 ergeben sich für das Lymphotoxin bzw. die LT-Mutanten folgende biologisiche Aktivitäten (angegeben werden diejenigen Protein-Konzentrationen, die eine 50 %ige Lyse einer gegebenen Zellzahl bewirken):

| Substanz | Proteinkonzentration $EC_{50}$ (ng Protein/ml) |
|---|---|
| LT | 0,55 |
| delta 23LT | 0,29 |
| delta 24LT | 0,15 |
| delta 25LT | 0,38 |

12. Synergistische zytotoxische Aktivität von humanem recombinantem Lymphotoxin und seinen Mutanten mit humanem recombinantem Interferon-gamma auf Tumorzellen.

Zum Nachweis der synergistischen Aktivität von Lymphotoxin oder den LT-Mutanten mit Interferon-gamma wurde das menschliche Osteosarkom MG-63 benutzt, das gegen Lymphotoxin allein nicht sensitiv ist, durch Interferon-gamma aber dosisabhängig wachstumsinhibiert wird.

Der Versuch wurde folgendermaßen durchgeführt:

$2x10^3$ sich im exponentiellen Wachstum befindliche Zellen wurden pro Vertiefung einer 96-Loch-Platte in komplettem Kulturmedium (RPMI 1640 + 10 % FCS, beides von Flow Laboratories, Meckenheim) ausplattiert und über Nacht bei 37°C, 5 % $CO_2$ in einer Wasserdampf-gesättigten Atmosphäre inkubiert. Danach wurden bei konstant gehaltenen Interferon-gamma Konzentrationen (rekombinant, menschlich) das Lymphotoxin oder die Lymphotoxinmutanten seriell 5-fach verdünnt, wobei die Startkonzentration 10 ng Protein/ml beträgt. Das Endvolumen beträgt in allen Kulturlöchern 150 $\mu$l. Als Kontrollen wurden reines Kulturmedium oder kultivierte Zellen ohne Zusatz von IFN-gamma und/oder LT bzw. LT-Mutanten verwendet. Die Kulturplatten wurden für weitere 72 h unter den oben angegebenen Bedingungen inkubiert und danach mittels der Kristallviolettfärbung, wie unter 11. beschrieben, angefärbt und photometrisch ausgewertet.

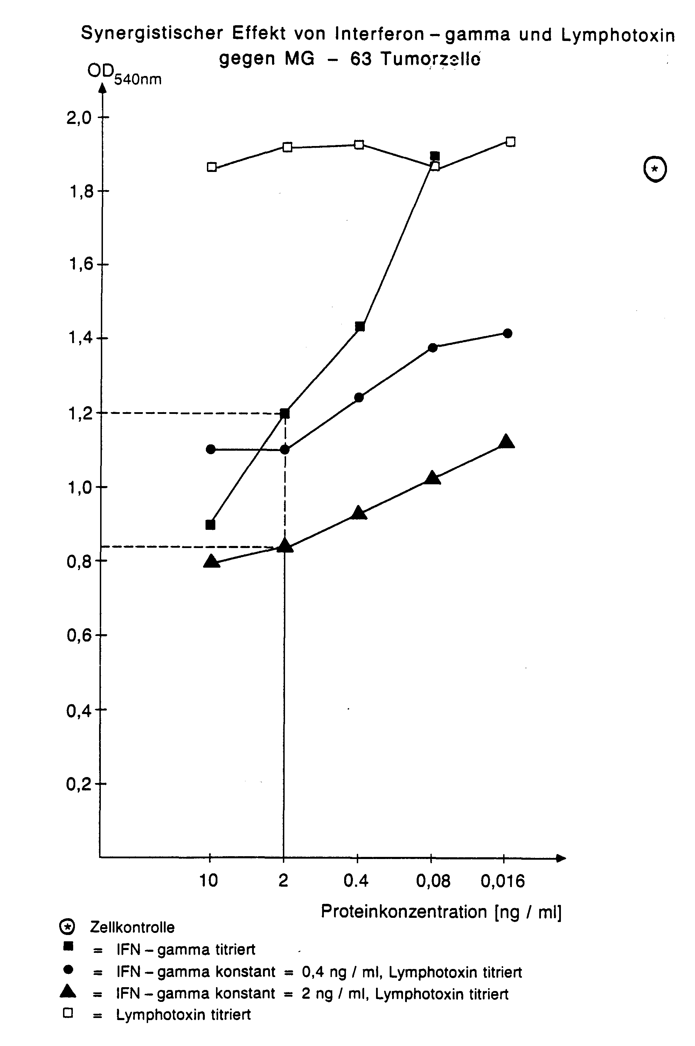

Die experimentellen Daten zur Bestimmung des synergistischen Effekts von Interferon-gamma und Lymphotoxin gegen die MG-63 Tumerzellinie sind in Abb. 7 dargestellt.

Die synergistische Wirkung von Lymphotoxinen mit Interferon-gamma wurde folgendermaßen bestimmt:

a) die antiproliferative Aktivität von Interferon-gamma wurde aus den optischen Dichten der Proben nach folgender Formel ermittelt:

$$\text{Aktivität INF-gamma} = 100\ \% - \frac{\text{OD 540 nm der behandelten Zellen}}{\text{OD 540 nm, der Zellkontrolle}} \times 100\ \% \quad (A)$$

Beispiel: 2 ng/ml IFN-gamma; siehe dazu Abb.7

$$\text{Aktivität IFN-gamma} = 100\ \% - \frac{1,20}{1,85} \times 100\ \% = 35\ \%.$$

b) Ermittlung der zytotoxischen Aktivität der Kombination Interferon-gamma mit Lymphotoxin, gemäß der Formel A.
Beispiel: 2 ng/ml Interferon-gamma und 2 ng/ml LT; siehe dazu Abb. 7

$$\text{Aktivität IFN gamma + LT} = 100\ \% - \frac{0,83}{1,85} \times 100\ \% = 55\ \%.$$

c) Bestimmung des synergistischen Effektes von Lymphotoxinen mit Interferon-gamma:

$$\text{Synergistischer Effekt} = \frac{\text{Aktivität IFN-}\gamma\text{+ LT}}{\text{Aktivität IFN-}\gamma}$$

Beispiel: 2 ng/ml IFN-gamma und 2 ng/ml Lymphotoxin; siehe dazu Abb. 7 und Beispiel 12a + b

$$\text{Synergistischer Effekt} = \frac{55\ \%}{35\ \%} = 1,57$$

Die Kombination von 2 ng/ml Interferon-gamma mit 2 ng/ml Lymphotoxin verstärkt den durch 2 ng/ml Interferon-gamma alleine induzierten zytotoxischen Effekt um den Faktor 1,57. Bei einer zweiten Messung wurde der Wert 1,41 erhalten. Der Mittelwert beider Zellen (1,49) ist in der Tabelle auf Seite 17 enthalten.

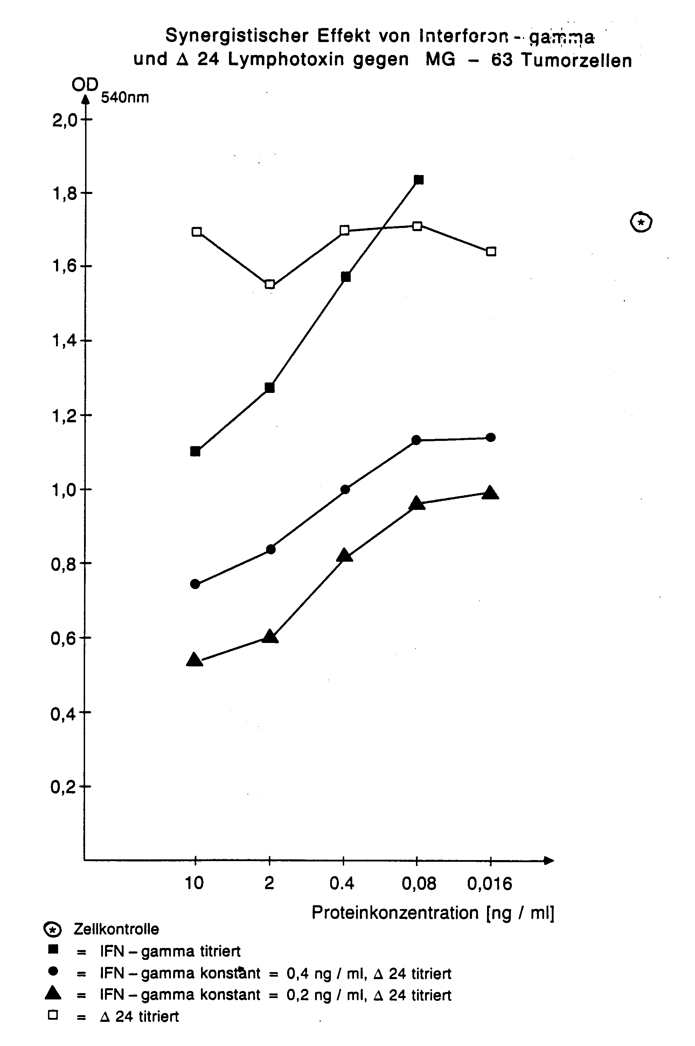

Die experimentellen Datan zur Bestimmung des synergistischen Effekts von Interferon-gamma und der Lymphotoxinmutante delta 24 gegen die MG-63 Tumorzellinie sind in Abb. 8 dargestellt. Die synergistische Wirkung von delta 24 LT mit Interferon-gamma wird wie vorher gezeigt bestimmt.

Die Ergebnisse zur Verstärkung der durch Interferon-gamma induzierten Zytotoxizität (synergistischer Effekt) durch die Kombination mit Lymphotoxin oder einem der hier beschriebenen Lymhotoxinmutanten sind in der folgenden Tabelle zusammengefaßt.

| LT-Spezies | IFN-gamma (ng/ml) | Eingesetzte Menge Lymphotoxin (ng/ml) | | | | |
|---|---|---|---|---|---|---|
| | | 10 | 2 | 0,4 | 0,08 | 0,016 |
| LT | 2,0 | 1,61 | 1,49 | 1,40 | 1,27 | 1,15 |
| | 0,4 | 2,16 | 1,89 | 1,75 | 1,87 | 1,42 |
| delta23 | 2,0 | 1,48 | 1,38 | 1,32 | 1,16 | 1,05 |
| | 0,4 | 2,0 | 1,90 | 1,62 | 1,27 | 1,24 |
| delta24 | 2,0 | 2,45 | 2,04 | 1,69 | 1,49 | 1,55 |
| | 0,4 | 6,59 | 3,75 | 2,69 | 1,96 | 2,37 |
| delta25 | 2,0 | 2,61 | 1,89 | 1,63 | 1,38 | 1,41 |
| | 0,4 | 8,30 | 3,45 | 3,04 | 1,45 | 1,65 |

Die in der Tabelle angegebenen Werte wurden, wie oben unter 12a bis 12c erläutert, ermittelt und stellen die Mittelwerte aus zwei unabhängigen Experimenten dar.

13. Bestimmung der Toxizität

Für die Bestimmung der akuten Toxizität der zu untersuchenden Substanzen wurden männliche Balb/c Mäuse im Alter von 4 bis 6 Wochen verwendet. Die Tiere wurden randomisiert (jeweils 5 Tiere pro Gruppe) und erhielten nach einer Akklimatisierungszeit von einer Woche die Testsubstanz in unterschiedlichen Dosierungen (0,25 mg/kg Körpergewicht - 4,0 mg/kg Körpergewicht intervenös in eine der lateralen Schwanzvenen injiziert. Das Applikationsvolumen betrug 10 ml/kg Körpergewicht; die Injektion erfolgte über einen Zeitraum von 10 Sekunden. Die Substanzen waren unmittelbar vor der intravenösen Injektion in physiologischer Kochsalzlösung, welche 0,2 % BSA (Bovine Serum Albumin) enthielt, gelöst worden. Als Kontrollen dienten Tiere, welche 10 ml physiologische Kochsalzlösung pro kg Körpergewicht mit 0,2 % BSA intravenös verabreicht bekamen. Nach Injektion der Testsubstanzen wurden sämtliche Tiere über einen Zeitraum von 7 Tagen beobachtet. Folgende Symptome wurden notiert: Diarrhoe, verringerte Lokomotion, Pilo-Erektion, Cyanose und Exitus; außerdem wurden regelmäßig die Körpertemperatur und das Körpergewicht der Tiere bestimmt.

Die Ergebnisse mehrerer Untersuchungen zeigen, daß sowohl Lymphotoxin (LT) als auch die Mutanten (delta 23LT, delta 24LT und delta 25LT) eine dosis-und zeitabhängige Verringerung der Körpertemperatur, des Körpergewichtes und der Lokomotion verursachten sowie eine Pilo-Erektion und Diarrhoe induzierten. Diese Symtome ließen sich bereits 2 Stunden nach der Injektion, vor allem bei höheren Dosierungen, nachweisen und waren in den über lebenden Tieren innerhalb der 7-tägigen Beaobachtungszeit voll reversibel. Die beobachteten Symptome waren in den Tieren, die mit LT behandelt worden waren am stärksten ausgeprägt. Tiere, welche mit den LT-Mutanten behandelt worden waren, zeigten eine insgesamt geringer ausgeprägte Symptomatik im Vergleich zum LT, wobei das delta 23LT aber deutlich stärkere Symtome verursachte als das delta 24LT; das delta 25LT nahme eine Mittelstellung zwischen dem delta 23LT und dem delta 24LT ein.

Die Schätzwerte für die jeweiligen $LD_{50}$-Werte wurden wie folgt berechnet:

$LD_{50}$ für LT : 0,79 mg/kg Körpergewicht
$LD_{50}$ für delta 23LT : 1,84 mg/kg Körpergewicht
$LD_{50}$ für delta 25LT : 3,1 mg/kg Körpergewicht
$LD_{50}$ für delta 24LT : nicht berechenbar, da unter keiner Dosierung ≥50 % der Tiere verstarben

**Patentansprüche**
**Patentansprüche für folgende Vertragsstaaten : BE, CH, DE, FR, GB, IT, LI, NL, SE**

**1.** Polypeptide der Formel

```
X-   LEU LYS PRO ALA ALA HIS LEU ILE GLY ASP PRO SER
LYS GLN ASN SER LEU LEU TRP ARG ALA ASN THR ASP ARG ALA PHE LEU
GLN ASP GLY PHE SER LEU SER ASN ASN SER LEU LEU VAL PRO THR SER
GLY ILE TYR PHE VAL TYR SER GLN VAL VAL PHE SER GLY LYS ALA TYR
SER PRO LYS ALA THR SER SER PRO LEU TYR LEU ALA HIS GLU VAL GLN
LEU PHE SER SER GLN TYR PRO PHE HIS VAL PRO LEU LEU SER SER GLN
LYS MET VAL TYR PRO GLY LEU GLN GLU PRO TRP LEU HIS SER MET TYR
HIS GLY ALA ALA PHE GLN LEU THR GLN GLY ASP GLN LEU SER THR HIS
THR ASP GLY ILE PRO HIS LEU VAL LEU SER PRO SER THR VAL PHE PHE
GLY ALA PHE ALA LEU
```

worin X für THR- oder SER-THR steht.

2. Verfahren zur Herstellung der Polypeptide nach Anspruch 1, dadurch gekennzeichnet, daß man
   a) aus einer Lymphotoxin produzierenden Zellinie die mRNA isoliert,
   b) diese mRNA in die entsprechende doppelsträngige cDNA copiert,
   c) diese cDNA in Vektoren von E.coli insertiert,
   d) E.coli mit den so erhaltenen neuen Vektoren transformiert,
   e) die Lymphotoxin cDNA Klone mit Hilfe von Gensonden und Hybridisierung selektioniert und charakterisiert,
   f) die das Lymphotoxin-Gen enthaltenden Vektoren vermehrt und isoliert,
   g) mit Hilfe von Restriktionsendonukleasen das Gen oder die Genfragmente isoliert,
   h) das Gen oder die Genfragmente mit geeigneten Oligonukleotiden in Expressionsvektoren insertiert,
   i) gegebenenfalls zusätzliche DNA-Sequenzen am 5' Ende der Gene insertiert,
   j) E. coli mit diesen Expressionsvektoren transformiert und
   k) das gewünschte Genprodukt exprimiert, isoliert und reinigt.

3. Vektoren, die Gensequenzen enthalten, die für die Polypeptide gemaß Anspruch 1 kodieren.

4. DNA-Sequenzen codierend für ein Polypeptid gemäß Anspruch 1.

5. Kombination aus einem Polypeptid gemäß Anspruch 1 und einem Lymphokin.

6. Arzneimittel enthaltend mindestens ein Polypeptid gemäß Anspruch 1.

7. Arzneimittel gemäß Anspruch 6, enthaltend eine Kombination aus mindestens einem Polypeptid gemäß Anspruch 1 und bekannten Lymphokinen und/oder Cancerostatica.

8. Polypeptide gemäß Anspruch 1 zur Verwendung bei der Bekämpfung von Krankheiten.

9. Kombination aus einem Polypeptid gemäß Anspruch 1 und einem Lymphokin und/oder Cancerostaticum zur Verwendung bei der Bekämpfung von Krankheiten.

**Patentansprüche für die Vertragsstaaten : AT, ES**

1. Verfahren zur Herstellung von Polypeptiden der Formel

```
X-  LEU LYS PRO ALA ALA HIS LEU ILE GLY ASP PRO SER
    LYS GLN ASN SER LEU LEU TRP ARG ALA ASN THR ASP ARG ALA PHE LEU
    GLN ASP GLY PHE SER LEU SER ASN ASN SER LEU LEU VAL PRO THR SER
    GLY ILE TYR PHE VAL TYR SER GLN VAL VAL PHE SER GLY LYS ALA TYR
    SER PRO LYS ALA THR SER SER PRO LEU TYR LEU ALA HIS GLU VAL GLN
    LEU PHE SER SER GLN TYR PRO PHE HIS VAL PRO LEU LEU SER SER GLN
    LYS MET VAL TYR PRO GLY LEU GLN GLU PRO TRP LEU HIS SER MET TYR
    HIS GLY ALA ALA PHE GLN LEU THR GLN GLY ASP GLN LEU SER THR HIS
    THR ASP GLY ILE PRO HIS LEU VAL LEU SER PRO SER THR VAL PHE PHE
    GLY ALA PHE ALA LEU
```

worin X für THR- oder SER-THR steht, dadurch gekennzeichnet, daß man

a) aus einer Lymphotoxin produzierenden Zellinie die mRNA isoliert,

b) diese mRNA in die entsprechende doppelsträngige cDNA copiert,

c) diese cDNA in Vektoren von E.coli insertiert,

d) E.coli mit den so erhaltenen neuen Vektoren transformiert,

e) die Lymphotoxin cDNA Klone mit Hilfe von Gensonden und Hybridisierung selektioniert und charakterisiert,

f) die das Lymphotoxin-Gen enthaltenden Vektoren vermehrt und isoliert,

g) mit Hilfe von Restriktionsendonukleasen das Gen oder die Genfragmente isoliert,

h) das Gen oder die Genfragmente mit geeigneten Oligonukleotiden in Expressionsvektoren insertiert,

i) gegebenenfalls zusätzliche DNA-Sequenzen am 5' Ende der Gene insertiert,

j) E. coli mit diesen Expressionsvektoren transformiert und

k) das gewünschte Genprodukt exprimiert, isoliert und reinigt.

**Claims**
**Claims for the following Contracting States : BE, CH, DE, FR, GB, IT, LI, NL, SE**

1. Polypeptides of the formula

```
X- Leu Lys Pro Ala Ala His Leu Ile Gly Asp Pro Ser Lys
   Gln Asn Ser Leu Leu Trp Arg Ala Asn Thr Asp Arg Ala Phe
   Leu Gln Asp Gly Phe Ser Leu Ser Asn Asn Ser Leu Leu Val
   Pro Thr Ser Gly Ile Tyr Phe Val Tyr Ser Gln Val Val Phe
   Ser Gly Lys Ala Tyr Ser Pro Lys Ala Thr Ser Ser Pro Leu
   Tyr Leu Ala His Glu Val Gln Leu Phe Ser Ser Gln Tyr Pro
   Phe His Val Pro Leu Leu Ser Ser Gln Lys Met Val Tyr Pro
   Gly Leu Gln Glu Pro Trp Leu His Ser Met Tyr His Gly Ala
   Ala Phe Gln Leu Thr Gln Gly Asp Gln Leu Ser Thr His Thr
   Asp Gly Ile Pro His Leu Val Leu Ser Pro Ser Thr Val Phe
   Phe Gly Ala Phe Ala Leu
```

wherein X is Thr- or Ser-Thr.

2. A process for the preparation of the polypeptides as claimed in claim 1, which comprises

a) isolation of the mRNA from a lymphotoxin-producing cell line,

b) making a corresponding double-stranded cDNA copy of this mRNA,

c) insertion of this cDNA into vectors of E. coli,

d) transformation of E. coli with the resulting new vectors,

e) selection and characterization of the lymphotoxin cDNA clones using gene probes and hybridization,

f) multiplication and isolation of the vectors containing the lymphotoxin gene,

g) isolation of the gene or gene fragments using restriction endonucleases,

h) insertion of the gene or gene fragments with suitable oligonucleotides into expression vectors,

i) where appropriate, insertion of additional DNA sequences at the 5' end of the genes,

j) transformation of E. coli with these expression vectors, and

k) expression, isolation and purification of the desired gene product.

3. Vectors which contain gene sequences which code for the polypeptides as claimed in claim 1.

4. DNA sequences coding for a polypeptide as claimed in claim 1.

5. A combination of a polypeptide as claimed in claim 1 and a lymphokine.

6. A pharmaceutical containing one or more polypeptides as claimed in claim 1.

7. A pharmaceutical as claimed in claim 6, containing a combination of one or more polypeptides as claimed in claim 1, and known lymphokines and/or carcinostatics.

8. Polypeptides as claimed in claim 1 for use for controlling diseases.

9. A combination of a polypeptide as claimed in claim 1 and of a lymphokine and/or carcinostatic for use for controlling diseases.

**Claims for the following Contracting States : AT, ES**

1. A process for the preparation of polypeptides of the formula

X- Leu Lys Pro Ala Ala His Leu Ile Gly Asp Pro Ser Lys
Gln Asn Ser Leu Leu Trp Arg Ala Asn Thr Asp Arg Ala Phe
Leu Gln Asp Gly Phe Ser Leu Ser Asn Asn Ser Leu Leu Val
Pro Thr Ser Gly Ile Tyr Phe Val Tyr Ser Gln Val Val Phe
Ser Gly Lys Ala Tyr Ser Pro Lys Ala Thr Ser Ser Pro Leu
Tyr Leu Ala His Glu Val Gln Leu Phe Ser Ser Gln Tyr Pro
Phe His Val Pro Leu Leu Ser Ser Gln Lys Met Val Tyr Pro
Gly Leu Gln Glu Pro Trp Leu His Ser Met Tyr His Gly Ala
Ala Phe Gln Leu Thr Gln Gly Asp Gln Leu Ser Thr His Thr
Asp Gly Ile Pro His Leu Val Leu Ser Pro Ser Thr Val Phe
Phe Gly Ala Phe Ala Leu

where X is Thr- or Ser-Thr, which comprises

a) isolation of the mRNA from a lymphotoxin-producing cell line,

b) making a corresponding double-stranded cDNA copy of this mRNA,

c) insertion of this cDNA into vectors of E. coli,

d) transformation of E. coli with the resulting new vectors,

e) selection and characterization of the lymphotoxin cDNA clones using gene probes and hybridization,

f) multiplication and isolation of the vectors containing the lymphotoxin gene,

g) isolation of the gene or gene fragments using restriction endonucleases,

EP 0 250 000 B1

h) insertion of the gene or gene fragments with suitable oligonucleotides into expression vectors,
i) where appropriate, insertion of additional DNA sequences at the 5' end of the genes,
j) transformation of E. coli with these expression vectors, and
k) expression, isolation and purification of the desired gene product.

**Revendications**
**Revendications pour les Etats contractants suivants : BE, CH, DE, FR, GB, IT, LI, NL, SE**

**1.** Polypeptides de formule

```
X-   LEU LYS PRO ALA ALA HIS LEU ILE GLY ASP PRO SER
LYS GLN ASN SER LEU LEU TRP ARG ALA ASN THR ASP ARG
ALA PHE LEU GLN ASP GLY PHE SER LEU SER ASN ASN SER
LEU LEU VAL PRO THR SER GLY ILE TYR PHE VAL TYR SER
GLN VAL VAL PHE SER GLY LYS ALA TYR SER PRO LYS ALA
THR SER SER PRO LEU TYR LEU ALA HIS GLU VAL GLN LEU
PHE SER SER GLN TYR PRO PHE HIS VAL PRO LEU LEU SER
SER GLN LYS MET VAL TYR PRO GLY LEU GLN GLU PRO TRP
LEU HIS SER MET TYR HIS GLY ALA ALA PHE GLN LEU THR
GLN GLY ASP GLN LEU SER THR HIS THR ASP GLY ILE PRO
HIS LEU VAL LEU SER PRO SER THR VAL PHE PHE GLY ALA
PHE ALA LEU
```

dans laquelle X est mis pour THR- ou SER-THR.

**2.** Procédé de préparation des polypeptides selon la revendication 1, caractérisé par le fait
a) qu'à partir d'une ligne cellulaire produisant de la lymphotoxine, on isole le mARN,
b) on copie ce mARN sur le cADN à double chaîne correspondant,
c) on fait s'intégrer ce cADN dans des vecteurs d'E. coli,
d) avec les nouveaux vecteurs ainsi obtenus on transforme E. coli,
e) on sélectionne les clones de cADN lymphotoxine à l'aide de sondes de gène et d'hybridation et on les caractérise,
f) on multiplie les vecteurs contenant le gène de lymphotoxine et on les isole,
g) à l'aide d'endonucléases de restriction on isole le gène ou les fragments de gène,
h) à l'aide d'oligonucléotides appropriés on fait s'intégrer le gène ou les fragments de gène dans des vecteurs d'expression,
i) à l'extrémité 5' des gènes on fait éventuellement s'intégrer des séquences d'ADN additionnelles,
j) avec ces vecteurs d'expression on transforme E. coli et
k) on exprime, isole et purifie le produit gène souhaité.

**3.** Vecteurs contenant les séquences de gène codant pour les polypeptides selon la revendication 1.

**4.** Séquences d'ADN codant pour un polypeptide selon la revnedication 1.

**5.** Combinaison d'un polypeptide selon la revendication 1 et d'une lymphokine.

**6.** Médicament contenant au moins un polypeptide selon la revendication 1.

**7.** Médicament selon la revendication 6, contenant une combinaison d'au moins un polypeptide selon la revendication 1 et de lymphokines et/ou cancérostatiques connus.

13

**8.** Polypeptides selon la revendication 1 pour l'utilisation dans la lutte contre des maladies.

**9.** Combinaison d'un polypeptide selon la revendication 1 et d'une lymphokine et/Ou d'un cancérostatique pour l'utilisation dans la lutte contre des maladies.

**Revendications pour les Etats contractants suivants : AT, ES**

**1.** Procédé de fabrication de polypeptides de formule

```
X-   LEU LYS PRO ALA ALA HIS LEU ILE GLY ASP PRO SER

LYS GLN ASN SER LEU LEU TRP ARG ALA ASN THR ASP ARG

ALA PHE LEU GLN ASP GLY PHE SER LEU SER ASN ASN SER

LEU LEU VAL PRO THR SER GLY ILE TYR PHE VAL TYR SER

GLN VAL VAL PHE SER GLY LYS ALA TYR SER PRO LYS ALA

THR SER SER PRO LEU TYR LEU ALA HIS GLU VAL GLN LEU

PHE SER SER GLN TYR PRO PHE HIS VAL PRO LEU LEU SER

SER GLN LYS MET VAL TYR PRO GLY LEU GLN GLU PRO TRP

LEU HIS SER MET TYR HIS GLY ALA ALA PHE GLN LEU THR

GLN GLY ASP GLN LEU SER THR HIS THR ASP GLY ILE PRO

HIS LEU VAL LEU SER PRO SER THR VAL PHE PHE GLY ALA

PHE ALA LEU
```

dans laquelle X est mis pour THR- ou SER-THR, caractérisé par le fait
a) qu'à partir d'une ligne cellulaire produisant de la lymphotoxine, on isole le mARN,
b) on copie ce mARN sur le cADN à double chaîne correspondant,
c) on fait s'intégrer ce cADN dans des vecteurs d'E. coli,
d) avec les nouveaux vecteurs ainsi obtenus on transforme E. coli,
e) on sélectionne les clones de cADN lymphotoxine à l'aide de sondes de gène et d'hybridation et on les caractérise,
f) on multiplie les vecteurs contenant le gène de lymphotoxine et on les isole,
g) à l'aide d'endonucléases de restriction on isole le gène ou les fragments de gène,
h) à l'aide d'oligonucléotides appropriés on fait s'intégrer le gène ou les fragments de gène dans des vecteurs d'expression,
i) à l'extrémité 5' des gènes on fait éventuellement s'intégrer des séquences d'ADN additionnelles,
j) avec ces vecteurs d'expression on transforme E. coli et
k) on exprime, isole et purifie le produit gène souhaité.

Abbildung 1

```
-34
MET  THR  PRO  PRO  GLU  ARG  LEU  PHE  LEU  PRO  ARG  VAL  CYS  GLY  THR  THR  LEU  HIS
                                                                                -1  +1
LEU  LEU  LEU  LEU  GLY  LEU  LEU  VAL  LEU  LEU  LEU  PRO  GLY  ALA  GLN  GLY  LEU  PRO
GLY  VAL  GLY  LEU  THR  PRO  SER  ALA  ALA  GLN  THR  ALA  ARG  GLN  HIS  PRO  LYS  MET
         +24  +25  +26
HIS  LEU  ALA  HIS  SER  THR  LEU  LYS  PRO  ALA  ALA  HIS  LEU  ILE  GLY  ASP  PRO  SER
LYS  GLN  ASN  SER  LEU  LEU  TRP  ARG  ALA  ASN  THR  ASP  ARG  ALA  PHE  LEU  GLN  ASP
GLY  PHE  SER  LEU  SER  ASN  ASN  SER  LEU  LEU  VAL  PRO  THR  SER  GLY  ILE  TYR  PHE
VAL  TYR  SER  PHE  VAL  GLY  SER  GLY  LYS  ALA  TYR  SER  SER  PRO  LYS  ALA  THR  SER
SER  PRO  LEU  TYR  ALA  HIS  GLU  GLN  VAL  GLN  LEU  PHE  SER  SER  GLN  TYR  PRO  PHE
HIS  VAL  PRO  LEU  SER  SER  GLN  LYS  MET  VAL  TYR  PRO  GLY  LEU  GLN  GLU  PRO  PRO
TRP  LEU  HIS  SER  MET  TYR  HIS  GLY  ALA  ALA  PHE  GLN  LEU  THR  GLN  GLY  ASP  GLN
LEU  SER  THR  HIS  ASP  GLY  ILE  PRO  HIS  LEU  VAL  LEU  SER  PRO  SER  THR  SER  VAL
               +171
PHE  PHE  GLY  ALA  PHE  ALA  LEU  LEU
```

Abb. 1: Aminosäuresequenz des Lymphotoxins mit Signalsequenz

Abbildung 2

```
LEU  PRO  GLY  VAL  GLY  LEU  THR  PRO  SER  ALA  ALA  GLN  THR  ALA  ARG  GLN  HIS  PRO
                                                                                      VAL

LYS  MET  HIS  LEU  ALA  HIS  SER  THR  LEU  LYS  PRO  ALA  ALA  HIS  LEU  ILE  GLY  ASP
ARG  SER  SER  SER  ARG  THR  PRO  SER  ASP  LYS  PRO  VAL  ALA  HIS  VAL  VAL  ALA  ASN

PRO  SER  LYS  GLN  ASN  SER  LEU  LEU  TRP  ARG  ALA  ASN  THR  ASP  ARG  ALA  PHE  LEU
PRO  GLN  ALA  GLU  GLY  GLN  LEU  GLN  TRP  LEU  ASN  ARG  ARG  ALA  ASN  ALA  LEU  LEU

GLN  ASP  GLY  PHE  SER  LEU  SER  ASN  ASN  SER  LEU  LEU  VAL  PRO  THR  SER  GLY  ILE
ALA  ASN  GLY  VAL  GLU  LEU  ARG  ASP  ASN  GLN  LEU  VAL  VAL  PRO  SER  GLU  GLY  LEU

TYR  PHE  VAL  TYR  SER  GLN  VAL  VAL  PHE  SER  GLY  LYS  ALA  TYR  SER  PRO  LYS  ALA
TYR  LEU  ILE  TYR  SER  GLN  VAL  LEU  PHE  LYS  GLY  GLN  GLY  ---  CYS  PRO  ---  ---

THR  SER  SER  PRO  LEU  TYR  LEU  ALA  HIS  GLU  VAL  GLN  LEU  PHE  SER  SER  GLN  TYR
---  SER  THR  HIS  VAL  LEU  LEU  THR  HIS  THR  ILE  SER  ARG  ILE  ALA  VAL  SER  TYR

PRO  PHE  HIS  VAL  PRO  LEU  LEU  SER  SER  GLN  LYS  MET  VAL  TYR  ---  ---  ---  ---
GLN  THR  LYS  VAL  ASN  LEU  LEU  SER  ALA  ILE  LYS  SER  PRO  CYS  GLN  ARG  GLU  THR

---  PRO  GLY  LEU  GLN  ---  GLU  PRO  TRP  LEU  HIS  SER  MET  TYR  HIS  GLY  ALA  ALA
PRO  GLU  GLY  ALA  GLU  ALA  LYS  PRO  TRP  TYR  GLU  PRO  ILE  TYR  LEU  GLY  GLY  VAL

PHE  GLN  LEU  THR  GLN  GLY  ASP  GLN  LEU  SER  THR  HIS  THR  ASP  GLY  ILE  PRO  HIS
PHE  GLN  LEU  GLU  LYS  GLY  ASP  ARG  LEU  SER  ALA  GLU  ILE  ASN  ARG  PRO  ASP  TYR

LEU  VAL  LEU  SER  PRO  SER  ---  THR  VAL  PHE  PHE  GLY  ALA  PHE  ALA  LEU
LEU  ASP  PHE  ALA  GLU  SER  GLY  GLN  VAL  TYR  PHE  GLY  ILE  ILE  ALA  LEU
```

Abb. 2: Vergleich der Aminosäuresequenzen von humanem Lymphotoxin (TNF-beta, obere Formel) und humanem Tumor Nekrose Faktor (TNF-alpha untere Formel) ohne Signalsequenzen.

EP 0 250 000 B1

Abbildung 3

Sau3A
GATCCCCGGC  CTGCCTGGGC  CTGGGCCTTG  GTTCTCCCCA  TGACACCACC  TGAACGTCTC  TTCCTCCCAA

GGGTGTGTGG  CACCACCCTA  CACCTCCTCC  TTCTGGGGCT  GCTGCTGGTT  CTGCTGCCTG  GGGCCCAGGG

GCTCCCTGGT  GTTGGCCTCA  CACCTTCAGC  TGCCCAGACT  GCCCGTCAGC  ACCCCAAGAT  GCATCTTGCC


     BbvI
CACAGCACCC  TCAAACCTGC  TGCTCACCTC  ATTGGAGACC  CCAGCAAGCA  GAACTCACTG  CTCTGGAGAG

CAAACACGGA  CCGTGCCTTC  CTCCAGGATG  GTTTCTCCTT  GAGCAACAAT  TCTCTCCTGG  TCCCCACCAG


       AccI
TGGCATCTAC  TTCGTCTACT  CCCAGGTGGT  CTTCTCTGGG  AAAGCCTACT  CTCCCAAGGC  CACCTCCTCC

CCACTCTACC  TGGCCCATGA  GGTCCAGCTC  TTCTCCTCCC  AGTACCCCTT  CCATGTGCCT  CTCCTCAGCT

CCCAGAAGAT  GGTGTATCCA  GGGCTGCAGG  AACCCTGGCT  GCACTCGATG  TACCACGGGG  CTGCGTTCCA

GCTCACCCAG  GGAGACCAGC  TATCCACCCA  CACAGATGGC  ATCCCCCACC  TAGTCCTCAG  CCCTAGTACT

GTCTTCTTTG  GAGCCTTCGC  TCTGTAGAAC  TTGGAAAAAT  CCAGAAAGAA  AAAATAATTG  ATTTCAAGAC

CTTCTCCCCA  TTCTGCCTCC  ATTCTGACCA  TTTCAGGGGT  CGTCACCACC  TCTCCTTTGG  CCATTCCAAC


          Sau3A
AGCTCAAGTC  TTCCCTGATC


Abb. 3: cDNA-Sequenz des humanen Lymphotoxin-Klons

Bam H I

Bbv I

Ori

Acc I

Lymphotoxin
c DNA

plyt 15

amp^R

(Bam H I) / Sau III A

Sal I

KS 27  A ATT C ATG AGC
KS 28      GTACTCGTGGG

Bbv I / Acc I Fragment
+ Acc I / Sal I Fragment

Eco R I     Acc I                                    Sal I

Bbv I

Eco R I

Ori

Sal I

puc18

amp^R

Eco R I / Sal I
großes Fragment

Eco R I

Ori

Acc I

Bbv I

pks 302

amp^R

Sal I

## Synergistischer Effekt von Interferon – gamma und Lymphotoxin gegen MG – 63 Tumorzelle

⊛  Zellkontrolle

■  =  IFN – gamma titriert

●  =  IFN – gamma konstant = 0,4 ng / ml, Lymphotoxin titriert

▲  =  IFN – gamma konstant = 2 ng / ml, Lymphotoxin titriert

□  =  Lymphotoxin titriert

## Synergistischer Effekt von Interferon - gamma
## und Δ 24 Lymphotoxin gegen  MG  – 63 Tumorzellen

- ⊛ Zellkontrolle
- ■ = IFN – gamma titriert
- ● = IFN – gamma konstant = 0,4 ng / ml, Δ 24 titriert
- ▲ = IFN – gamma konstant = 0,2 ng / ml, Δ 24 titriert
- □ = Δ 24 titriert